# EUROPÄISCHE PATENTSCHRIFT

(11) **EP 0 592 750 B1**
(45) Veröffentlichungstag und Bekanntmachung des Hinweises auf die Patenterteilung: **27.01.1999**
(21) Anmeldenummer: 92810957.8
(22) Anmeldetag: 04.12.1992
(51) Int. Cl.: A61F 2/38

(54) **Meniskusplattform zu künstlichem Kniegelenk**
Meniscus platform for artificial knee joint
Plate-forme méniscale pour articulation artificelle du genou

(30) Priorität: 14.01.1992 CH 89/92
(43) Veröffentlichungstag der Anmeldung: 20.04.1994
(73) Patentinhaber: Sulzer Orthopädie AG, 6340 Baar (CH)
(72) Erfinder: Rorabeck, Cecil H., Dr., London, Ontario N6G 2K1 (CA); Bourne, Robert B., Dr., London, Ontario N6K 3S6 (CA)
(74) Vertreter: Sulzer Management AG

(56) Entgegenhaltungen:
- EP-A- 0 186 471
- WO-A-89/09578
- DE-A- 2 660 623
- FR-A- 2 576 793
- FR-A- 2 663 536
- US-A- 4 224 696
- US-A- 4 936 853

## Beschreibung

Die Erfindung handelt von einer Meniskusplattform zu künstlichem Kniegelenk gemäß des Oberbegriffes des Anspruchs 1.

Die Problematik und Lösungsformen für künstliche Kniegelenke sind ausführlich in US-PS 4,309,778 beschrieben. Die gezeigten Lösungsformen probieren den Bewegungsmechanismus des natürlichen Kniegelenks nachzuahmen und möglichst sicher in Bezug auf die Führung der beweglichen Elemente zu gestalten. Sie beanspruchen einen für die Sicherung der Führungsbewegung entsprechend grossen Raumanteil, um den eine Resektion im Knochengewebe des Tibiaknochens vorgenommen werden muss.

Die FR-A-2 663 536 zeigt eine Kniegelenkprothese, deren bewegliches Zwischenteil mit einem Langloch längs einem Schraubenkopf auf einer Tibiaplattform verschiebbar geführt ist, wobei die Drehung gegenüber der Tibiaplattform durch Festanschläge begrenzt ist und in bestimmten Stellungen eine Drehung ausgeschlossen ist. Das Langloch im Zwischenteil besitzt eine obere Ansekung, in welche der Senkkopf einer in der Plattform eingedrehten Halteschraube zur Führung eingreift und das Zwischenteil gegen ein Abheben sichert.

Aufgabe der vorliegenden Erfindung ist es, die vorher beschriebene Kniegelenkprothese weiterzubilden, ohne zu stark vom Bewegungsablauf des natürlichen Kniegelenks abzuweichen. Diese Aufgabe wird mit den kennzeichnenden Merkmalen von Anspruch 1 gelöst. Die Erfindung hat den Vorteil, dass durch den geringen Resektionsanteil das hintere Kreuzband und auch die Seitenbänder erhalten bleiben können und sich an der Wegbegrenzung für bewegliche Elemente ähnlich dem natürlichen Knie beteiligen und somit für die Führung der beweglichen Elemente untereinander weniger mechanische Anschläge notwendig sind. Die abhängigen Unteransprüche 2 bis 6 beziehen sich auf vorteilhafte Weiterbildungen der Erfindung.

Im folgenden wir die Erfindung von einem Ausführungsbeispiel beschrieben. Es zeigen:

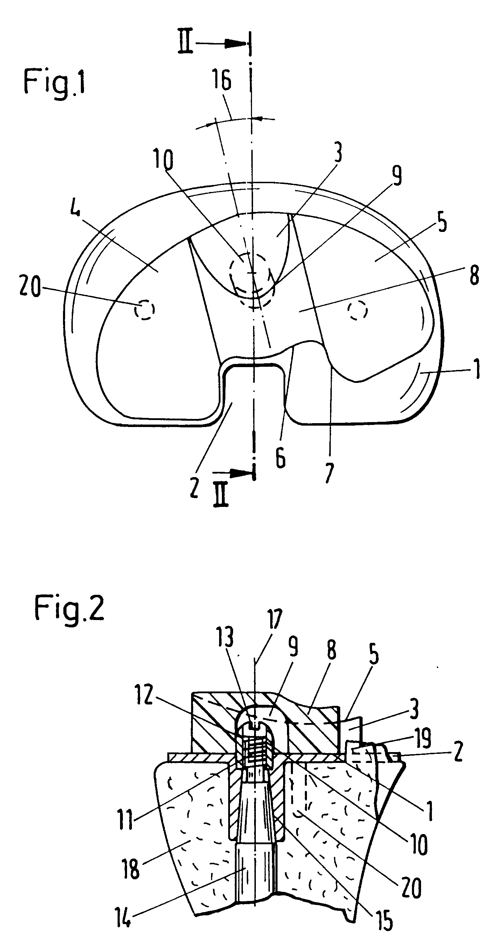

- Fig. 1: Schematisch die Draufsicht auf eine metallische Plattform mit Führungszapfen und mit aufgelegtem Kunststoffkörper und
- Fig. 2: schematisch einen Vertikal schnitt durch eine Meniskusplattform gemäss Fig. 1.

In den Figuren ist eine Meniskusplattform zu einem künstlichen Kniegelenk gezeigt, die aus einer metallischen Plattform 1 und einem quer zur Tibiaachse 17 darauf gleitenden Kunststoffkörper 3 besteht, der auf seiner Oberseite Gleitflächen 4, 5 und einen Führungswulst 8 für Kondylen der Femurseite aufweist. Die Gleitbewegung zwischen Kunststoffkörper 3 und metallischer Plattform 1 ist dabei durch einen Führungszapfen 10 in der metallischen Plattform, der in ein Langloch 9 im Kunststoffkörper 3 eingreift, begrenzt. Der Kunststoffkörper 3 kann sich um den Zapfen 10 drehen und längs seinem Langloch 9 verschoben werden. Die Drehbewegung des Kunststoffkörpers 3 wird durch eine begrenzende Aussparung 6, die einen Teil der Eminentia 19 mit dem hinteren Kreuzband einschliesst begrenzt.

Die metallische Plattform 1 ist mit zwei Verankerungselementen 20 in Form von Stiften und mit einem Verankerungszapfen 14 in der Tibia 18 in Richtung der Tibiaachse 17 verankert. Der Verankerungszapfen 14 endet an der Plattform als Gewindestück und ist mit einer als Führungszapfen 10 ausgebildeten Kontermutter 12 zu einer Bewegungssicherung 11 verschraubt. Zum Einsetzen und Befestigen eines in der Länge passenden Verankerungszapfen 14 kann die mit einem Schlitz 13 versehene Kontermutter 12 mit einem Hilfswerkzeug angezogen werden und eine starre konische Verbindung 15 zwischen Verankerungszapfen 14 und metallischer Plattform 1 bewirken. Der Kunststoffkörper 3 hat kleinere Seitenabmessungen als die metallische Plattform 1, damit er bei einer Schwenkbewegung um den Führungszapfen 10 nicht über die Plattform hervorsteht. Er besitzt auf seiner Unterseite in der Mittelebene vom Führungswulst 8 ein Langloch 9, das eine begrenzte und geführte Gleitbewegung in der Richtung dieser Mittelebene auf der Plattform zulässt. Die Drehbewegung um den Führungszapfen 10 ist bis zu einem Winkel 16 von 15° aus der Mittellage möglich und wird von einem W-förmig begrenzenden Ausschnitt 6, der grösser als der Ausschnitt 2 der metallischen Plattform 1 ist, begrenzt, wenn der begrenzende Ausschnitt 6 mit einer seiner Seitenwangen 7 am stehengebliebenen Teil der Eminentia 19 anstösst. Innerhalb dieser mechanischen Begrenzungen ergibt sich die Lage des Kunststoffkörpers 3 als Gleichgewichtszustand aus den von den Kondylen der Femurseite übertragenen Gewichtskräften und den von den Bändern zwischen Femur und Tibia erzeugten Kräften. Die Gleitflächen 4, 5 am Kunststoffkörper sind wannenartig vertieft und richten sich nach den Kondylen aus solange eine Anpresskraft vorhanden ist. Mit nachlassender Anpresskraft übernimmt der Führungswulst die Ausrichtung nach den Kondylen.

### Interne Stückliste

- 1: metallische Plattform
- 2: Aussparung
- 3: Kunststoffkörper
- 4: Gleitfläche
- 5: Gleitfläche
- 6: begrenzende Aussparung
- 7: Seitenwange
- 8: Führungswulst
- 9: Langloch
- 10: Führungszapfen
- 11: Bewegungssicherung
- 12: Kontermutter
- 13: Schlitz
- 14: Verankerungszapfen
- 15: konische Verbindung
- 16: Winkel
- 17: Tibiaachse
- 18: Tibia
- 19: Eminentia
- 20: Verankerungselement

## Patentansprüche

1. Meniskusplattform zu künstlichem Kniegelenk mit einer metallischen Plattform (1), welche eine Aussparung (2) für das hintere Kreuzband und stehengelassene Teile der Eminentia (19) aufweist und auf welcher ein Kunststoffkörper (3) mit einem Langloch (9) durch einen mit der Plattform (1) verschraubten Führungszapfen (10) eine geführte Längsbewegung und eine Drehbewegung ausführen kann, wobei der Kunststoffkörper (3) auf seiner Oberseite Gleitflächen (4, 5) für Kondylen der Femurseite aufweist,
dadurch gekennzeichnet, dass der Kunststoffkörper (3) auf seiner Oberseite einen Führungswulst (8) für die Kondylen der Femurseite aufweist, in welchem von unten das Langloch (9) als rundum geschlossenes Sackloch mit zylindrischen Seitenflächen eingelassen ist, und dass das Langloch (9) in beiden Richtungen der Längsbewegung an dem Führungszapfen (10), der kreiszylindrische Führungsflächen aufweist, anschlägt, wobei in allen Längsstellungen des Kunststoffkörpers (3) durch eine begrenzende Aussparung (6) im Kunststoffkörper (3) eine durch die Eminentia (19) im Bereich des hinteren Kreuzbandes beschränkte Schwenkbewegung um den Führungszapfen (10) zugelassen ist, die ein Ausschwenken aus der Mittellage bis zu einem Winkel (16) gestattet, ohne dass eine Ueberdeckung der Aussparung (2) stattfindet.

2. Meniskusplattform nach Anspruch 1, dadurch gekennzeichnet, dass das Langloch (9) im nicht geschwenkten Kunststoffkörper (3) in sagittaler Richtung verläuft.

3. Meniskusplattform nach Anspruch 1 oder 2, dadurch gekennzeichnet, dass der Führungszapfen (10) eine von oben zugängliche Bewegungssicherung (11) für einen von unten an der metallischen Plattform eingesetzten Verankerungszapfen (14) bildet.

4. Meniskusplattform nach einem der Ansprüche 1 bis 3, dadurch gekennzeichnet, dass der Winkel (16) zwischen 5° und 20° beträgt.

5. Meniskusplattform nach einem der Ansprüche 1 bis 4, dadurch gekennzeichnet, dass die metallischen Gleitflächen vom Führungszapfen (10) und die der metallischen Plattform (1) zum Kunststoffkörper (3) eine Gleitschicht aus Titan Nitrid aufweisen.

6. Meniskusplattform nach Anspruch 3, dadurch gekennzeichnet, dass der Führungszapfen aus einer Schraubenmutter (12) besteht, die den Verankerungszapfen (14) gegen die Plattform (1) sichert.

## Claims

1. A meniscus platform for an artificial knee joint with a metallic platform (1), which has a recess (2) for the posterior cruciate ligament and remaining sections of the eminence (19) and on which a synthetic component (3) with an elongated slot (9) can perform guided longitudinal motion and rotational motion by means of a guide pin (10) screwed to the platform (1), wherein the synthetic component (3) has slide planes (4, 5) on the upper face for condyles on the face of the femur,
characterised in that the synthetic component (3) has a guide bulge (8) on its upper face for the condyles on the face of the femur, the elongated slot (9) being set into this from below as a totally enclosed pocket hole with cylindrical lateral sides, and that the elongated slot (9) impinges, in both directions of longitudinal motion, on the guide pin (10) which has circular cylindrical guide faces, wherein, for all longitudinal positions of synthetic component (3), swivelling motion is permitted about the guide pin (10), restricted by the eminence (19) in the region of the posterior cruciate ligament, which allows an outwards swing from the mid-position to an angle (16) without the recess (2) being covered up, due to the presence of limiting recess (6) in synthetic component (3).

2. Meniscus platform according to Claim 1, characterised in that the elongated slot (9) runs in the sagittal direction when the synthetic component (3) is in the non-swivelled position.

3. Meniscus platform according to Claim 1 or 2, characterised in that the guide pin (10) forms a movement locking device (11), which is accessible from above, for anchorage pin (14) inserted into the metallic platform from below.

4. Meniscus platform according to one of Claims 1 to 3, characterised in that the angle (16) is between 5° and 20°.

5. Meniscus platform according to one of Claims 1 to 4, characterised in that the metallic slide planes of guide pin (10) and of metallic platform (1) to synthetic component (3) have a slip layer made of titanium nitride.

6. Meniscus platform according to Claim 3, characterised in that the guide pin consists of a screw nut (12) which secures anchorage pin (14) against the platform (11).

## Revendications

1. Plate-forme méniscale pour articulation artificielle du genou avec une plate-forme métallique (1) qui présente un évidement (2) pour le ligament croisé arrière et des parties qui subsistent de l'éminence (19) et sur laquelle un corps en matière synthétique (3) avec un trou oblong (9), par un tenon de guidage (10) assemblé par vissage avec la plate-forme (1) peut exécuter un mouvement longitudinal et un mouvement de rotation guidé, le corps en matière synthétique (3) présentant sur son côté supérieur des surfaces de glissement (4, 5) pour des condyles du côté fémur, caractérisée
en ce que le corps en matière synthétique (3) présente sur son côté supérieur un bourrelet de guidage (8) pour les condyles du côté fémur dans lequel est ménagé depuis en dessous le trou oblong (9) sous forme de trou borgne fermé tout autour avec des surfaces latérales cylindriques, et en ce que le trou oblong (9) bute dans les deux directions du mouvement longitudinal au tenon de guidage (10) qui présente des surfaces de guidage cylindriques circulaires où dans toutes les positions longitudinales du corps en matière synthétique (3), par un évidement de délimitation (6) dans le corps en matière synthétique (3), il est autorisé un mouvement de pivotement, limité par l'éminence (19) au voisinage du ligament croisé arrière, autour du tenon de guidage (10) qui permet un pivotement hors de la position médiane jusqu'à un angle (16) sans qu'il y ait un recouvrement de l'évidement (2).

2. Plate-forme méniscale selon la revendication 1, caractérisée en ce que le trou oblong (9), dans le corps en matière synthétique (3) non pivoté, s'étend dans la direction sagittale.

3. Plate-forme méniscale selon la revendication 1 ou 2, caractérisée en ce que le tenon de guidage (10) constitue une sécurité de mouvement (11) accessible depuis au-dessus pour un pivot d'ancrage (14) placé depuis en dessous dans la plate-forme métallique.

4. Plate-forme méniscale selon l'une des revendications 1 à 3, caractérisée en ce que l'angle (16) est compris entre 5° et 20°.

5. Plate-forme méniscale selon l'une des revendications 1 à 4, caractérisée en ce que les surfaces de glissement métalliques du tenon de guidage (10) et celles de la plate-forme métallique (1) vers le corps en matière synthétique (3) présentent une couche de glissement en nitrure de titane.

6. Plate-forme méniscale selon la revendication 3, caractérisée en ce que le tenon de guidage est constitué d'un écrou (12) qui assure le pivot d'ancrage (14) à l'encontre de la plate-forme (1).
